# NEW EUROPEAN PATENT SPECIFICATION

(11) **EP 2 421 504 B2**
(45) Date of publication and mention of the opposition decision: **10.08.2022**
(45) Mention of the grant of the patent: 08.03.2017
(21) Application number: 10717263.7
(22) Date of filing: 12.04.2010
(51) Int. Cl.: A61K 9/00, A61K 31/13, A61K 9/08

(54) **PAEDIATRIC SOLUTIONS COMPRISING PROPRANOLOL**
PÄDIATRISCHE LÖSUNGEN MIT PROPRANOLOL
SOLUTIONS PÉDIATRIQUES CONTENANT DU PROPRANOLOL

(30) Priority: 21.04.2009 EP 09290298
(43) Date of publication of application: 29.02.2012
(60) Divisional application: 12155472.9
(73) Proprietor: Pierre Fabre Dermatologie, 92100 Boulogne Billancourt (FR)
(72) Inventor: CHAUMONT, Christine, 92100 Boulogne-Billancourt (FR); CORDOLIANI, Jean-François, 92100 Boulogne Billancourt (FR); LEVERD, Elie, 92100 Boulogne Billancourt (FR); MUGUET, Valérie, 92100 Boulogne Billancourt (FR)
(74) Representative: Novagraaf Technologies
(86) International application number: PCT/IB2010/051573
(87) International publication number: WO 2010/122442

(56) References cited:
- DE-A1-102006 020 604
- GB-A- 2 264 866
- US-A- 4 600 708
- C.LEAUTE; E. DUMAS DE LA ROQUE; T: HUBICHE; F: BORALEVI; JB THAMBO; A. TAIEB: "propranolol for severe hemangiomas of infancy" N ENG JOURNAL OF MEDECINE, vol. 358, no. 24, 12 June 2008 (2008-06-12), pages 2649-2651, XP002546760
- TAKAHASHI N., MUROTA H., SUTOH I.: "Antimicrobial activity of topical beta-adrenergic blocking agents" OPHTALMIC RES., vol. 15, no. 6, 1983, pages 277-279, XP008112326
- Bonifazi E et al: "Propranolol in rapidly growing hemangiomas", EUROPEAN JOURNAL OF PEDIATRIC DERMATO,, vol. 18, no. 3, July 2008 (2008-07), pages 185-192,

## Description

### FIELD OF THE INVENTION

The present invention relates to pharmaceutical solutions and more particularly to paediatric solutions comprising propanolol or a pharmaceutically acceptable salt thereof, specifically an aqueous ethyl alcohol free propranolol solution comprising a non sugar type sweetener and less than 0.01 w/V% of any preservative agents, for use as a medicament in the treatment of hemangiomas, wherein the solution is a multi-use solution.

### BACKGROUND OF THE INVENTION

Infantile capillary hemangiomas (IH) in children are commonly recognized in the skin and liver, and represent most common soft tissue tumours in 4 to 10% of children aged under 1 year. Despite their begnin self-limited course, common IH are rarely responsible during their proliferative phase for local complications such as ulceration or haemorrhage but may however impair in some cases vital or sensorial functions especially when present respectively on upper airways and orbital areas.

While the understanding of growth and involution of IH is still an issue, it has however been found an alternative treatment to the oral corticosteroids, vincristine or interferon alfa-1a and 2b, thereby avoiding constraining side effects in young patients. Because patients are infants or small children, patient tolerance to treatments is of the utmost importance.

As it was reported by C. Léauté-Labrèze et al. in Propranolol for severe hemangiomas of infancy, N. Engl. J. Chem. (2008) 358; 24: 2649-51, it has been observed and demonstrated that beta-blockers, e.g. propranolol, could efficiently be used to control and even to treat the growth of IH.

Propranolol, (1-(isopropylamino)-3-(naphthalen-1-yloxy)propan-2-ol), is a well tolerated non-selective beta blocker commonly used in young children for cardiologic indications as disclosed in several publication e.g by Villain et al. in Low incidence of cardiac events with beta-blocking therapy in children with long QT syndrome, Eur Heart J. 2004, 25:1405-11; by Fritz et al in Effect of beta-blockade on symptomatic dexamethasone-induced hypertrophic obstructive cardiomyopathy in premature infants: three case reports and literature review. J. Perinatol 1998; 18:38-44 and by Kilian K. in Hypertension in neonates causes and treatments, J Perinat Neonatal Nurs, 2003; 17:65-74.

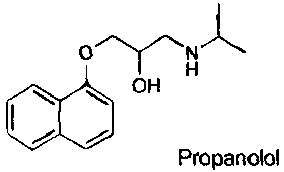

Propranolol is particularly available on the market under different formulations in the Syprol^{®} product, the Avlocardyl^{®} product or the drinkable solution of Roxane Laboratories. However, the existing solutions are not appropriate for a paediatric use. For example, the solution of Roxanne Laboratories contains alcohol (0.6%) which is particularly toxic in young patients.

Several aqueous solutions comprising propranolol have already been disclosed. In FR 2 688 405 for example, sustained release formulations for the oral delivery of beta blockers such as propranolol are prepared with polysaccharides and further comprise a preservative agent such as parabens. US 4,600,708 discloses liquid therapeutic dosage formulations of propranolol HCl containing lecithin and all of the disclosed examples contain methyl- and propylparaben. EP 732 064 relates to a bitterness-relieving agent which comprises an ester of a mono or diglyceride with a polycarboxylic acid salt. It is used to mask propranolol HCl in a liquid solution but however also encompasses a butyl-parabens excipient. Such preservative agents are not appropriate for a paediatric use.
Extemporaneous solutions of propranolol are also known. These were prepared by dissolving crushed tablets of propranolol in aqueous solutions together with preservative agents such as parabens or aromatic type preservatives. Indeed, their use is usually recommended as reported by D. Woods in "Extemporaneous formulations of Oral Liquids, a guide". Other known extemporaneous solutions for the oral administration of propranolol are syrup types which are thus based on a sucrose vehicle, which cannot be adapted for paediatric patients. Such syrups are provided for example in "Formulation in Pharmacy Practice" 2nd ed (www.pharminfotech.co.nz), or by V. Das Gupta in "Stability of Propranolol Hydrochloride Suspension and solution compounded from injection or tablets", Am. J. of Hospital Pharmacy, (1987) 44:360-361.
However, while numerous formulations of beta-blockers already exist in the art, none of them are suitable for paediatric patients since they contain alcoholic excipients or aromatic preservative agents such as benzyl alcohol that need to be carefully evaluated in young patients up to three years old. Furthermore, it has also been established that benzoic acid, sodium benzoate and potassium benzoate may increase the risk of jaundice in neonates. Similarly, ethanol is to be excluded. Thus, because aromatic preservatives and alcoholic excipients must not be given to neonates and young infants due to their immature metabolism, there has always been a need to provide safe and suitable compositions or formulations for the paediatric administration of beta-blockers.

### SUMMARY OF THE INVENTION

According to one aspect, the present invention is directed to an aqueous ethyl alcohol free paediatric solution comprising propranolol or pharmaceutically acceptable salts thereof, a non sugar type sweetener and less than 0.01 w/V% of any preservative agents, other than propranolol itself, for use as a medicament in the treatment of hemangiomas, wherein the solution is a multi-use solution.

According to one embodiment, the at least one non-sugar type sweetener is selected from saccharin, saccharin salts, sodium saccharin, calcium saccharin, sucralose, potassium acetosulfam, stevioside, steviol, mannitol, erythritol, lactitol, maltitol, alitame, miraculin, monellin, thaumatin, and mixture thereof, and for example selected from saccharin sodium.

According to one embodiment, the non-sugar type sweetener is in an amount of 0.05 to 0.5% w/V.

According to one embodiment, the beta-blocker propranolol is present in an amount of from 0.01 to 5% w/V, for example 0.01 to 1% w/V.

According to one embodiment, the aqueous solution further comprises at least one flavouring agent and/or at least one viscosity increasing agent.

According to one embodiment, the at least one non-aromatic preservative agent is selected from chlorobutanol, propionic acid or pharmaceutically acceptable salts thereof, sorbic acid or pharmaceutically acceptable salts thereof, and mixtures thereof, for example in an amount of less than 0.01 wN%.

According to one embodiment, the at least one flavouring agent is selected from any of the cherry, lemon, lime, mandarin, orange, tangerine, mint, strawberry, banana, caramel, liquorice, passion-fruit, peach, raspberry, tutti-frutti, grapefruit, vanilla, cream, chocolate, grape flavour or mixture thereof, for example selected from the vanilla and strawberry flavour, and for example in an amount of from 0 to 5% w/V, e.g. of from 0.01 to 1% w/V, e.g. of from 0.01 to 0.5% w/V and e.g. of from 0.05 to 0.5 % w/V.

According to one embodiment, the flavour is vanilla in an amount of from 0.01 to 0.5% w/V. According to one embodiment, the at least one viscosity increasing agent is selected from cellulose derivatives, hydroxyethylcellulose, hydroxypropylcellulose, hydroxypropylmethylcellulose, or methylcellulose, poloxamers, from gums, guar gum, tragacanthe gum, acacia gum, xanthane gum, gelane gums, alginic derivatives, alginic acid, sodium alginate, polyvinylpyrrolidone, from silicates, bentonite, laponite, veegum, and more particularly from non-ionic poloxamers, polyvinylpyrrolidone and cellulose ethers, for example hydroxyethylcellulose, and is for example in an amount of from 0 to 15% w/V, e.g. of from 0.1 to 10% w/V, e.g. of from 0.1 to 5 % w/V and e.g. of from 0.1 to 0.5%w/V.

According to one embodiment, propranolol is in an amount of from 0.01 to 20% w/V, for example of from 0.05 to 10% w/V, e.g. of from 0.01 to 5 % w/V, e.g. of from 0.01 to 1% w/V, of from 0.1 to 5 % w/V and e.g. of from 0.1 to 1% w/V, less than 0.01 w/V% of any preservative agents, other than propranolol itself, the at least one non-sugar type sweetener is in an amount of from 0.01 to 5% w/V, for example of from 0.05 to 1% w/V, e.g. of from 0.05 to 0.5 % w/V and e.g. of from 0.05 to 0.2%w/V, the at least one flavouring agent is in an amount of from 0 to 5% w/V, for example of from 0.05 to 1% w/V and e.g. of from 0.05 to 0.5 % w/V, the at least one viscosity increasing agent is in an amount of from 0 to 15% w/V, for example of from 0.1 to 10% w/V, e.g. of from 0.1 to 5 % w/V and e.g. of from 0.1 to 0.5%w/V.

According to one embodiment, the beta-blocker is propranolol hydrochloride in an amount of 0.428% or 0.57 % w/V, the at least one sweetener is saccharin sodium in an amount of 0.15% w/V, the at least one flavouring agent is a mixture of vanilla and strawberry flavour in an amount of 0.32% w/V the at least one viscosity increasing agent is hydroxyethylcellulose in an amount of 0.35 % w/V, and optionally comprising sodium propionate in an amount of less than 0.01 w/V%.

According to one embodiment of the present invention, the solution further comprises a pH regulator agent or buffer the pH is comprised between 2 and 6, for example 2 and 5.5, e.g., e.g. between 3.0 and 5.0 , e.g. between 2.0 to 5, e.g. 2.5 and 4.

According to one embodiment, propanolol is present in an amount of 0.250 to 1% w/V and vanilla is present in an amount of 0.01 to 1% w/V and the solution does not contain a preservative agent.

According to one embodiment, propanolol is present in an amount of 0.01 to 1% w/V, for example in an amount of 0.250 to 1% w/V and the at least flavouring agent is present in an amount of 0.01 to 1% w/V and the solution does contain a preservative agent in an amount of less than 0.01 w/V%. for example chlorobutanol or sodium propionate.

According to the present invention, the solution is a multi-use solution.

According to one embodiment, the solution is suitable for oral administration.

According to another aspect, the present invention is directed to a process for the preparation of the aqueous solutions of the invention, comprising the step of dissolving the propranolol into a solvent, for example water.

The present invention is directed to a solution of the invention for use as a medicament in the treatment of hemangiomas, for example capillary hemangiomas and e.g. capillary infantile hemangiomas.

The formulation of the present invention has the surprising advantage that when the beta-blocker propanolol is used, the addition of any other preservative can be avoided. It is known from Takahashi et al. Ophtalmic Res. 15, 277-279 published in 1983 that only bupranolol showed antimicrobial effects with a minimum inhibitory concentration of 0.1% on the growth of B. Subtilis and 0.05% on S. Aureus and E. Coli. Timolol and befunolol do not have these antimicrobial effects.

According to another aspect, the present invention is directed to the use of propranolol as a preservative agent.

According to another aspect, the invention is directed to the use of propanolol with vanilla flavour as a preservative agent.

According to another aspect, a device comprising a container and a scale marked pipette or oral syringe indicating doses based on the body weight of the patient, containing the solution of the invention is disclosed.

According to one embodiment, the scale marks each 0.1 ml doses.

### DETAILLED DESCRIPTION OF THE INVENTION

It has been found in a surprising manner that beta-blockers such as propranolol provide a preservative effect that could be used in formulations intended for paediatric patients. While conventional aromatic preservative agents should be excluded from paediatric products due to their toxic effects on immature metabolisms, these products should also fulfil the preservation requirements against micro-organism proliferation. This has been achieved in the present invention, based on the surprising discovery that beta-blockers such as propranolol provide suitable preservative and stability properties to aqueous solutions that are suitable for oral administration to young patients.

The solutions of the present invention are pharmaceutically acceptable and will be particularly adapted for multiple uses, having appropriate storage capabilities.

A most useful application is found in the treatment of infantile hemangiomas.

By 'pharmaceutically acceptable' is meant a material that is not biologically or otherwise undesirable, e.g. the material may be incorporated into a dosage form of the invention without causing any undesirable biological effects or interacting in a deleterious manner with any of the other components of the dosage form formulation.

As given in the recommendations of the reflection paper of the Committee for medicinal products for human use at the EMEA (EMEA/CHMP/PEG/194810/2005), oral administration is commonly used for paediatric patients, and liquid formulations are best suited for administration to young infants who are unable to swallow capsules or tablets.

Such aqueous solutions also include suspensions or emulsions that would fall within the scope of the present invention. They may be applied by instillation, spraying, squirting into the oral cavity of the patient, or mixed with milk or fruit juice before administration.

The term 'beta-blockers', refers to beta-receptor blocking agent, beta adrenergic receptor blocking agent, beta blocking agent, beta-blocking agent, beta-blocking agent or beta-adrenergic receptor blocking agent or any other denomination indicating a chemical that inhibits the binding of agonists, natural or artificial, to beta-adrenergic receptors of any type (bet-1, beta-2, beta-3 or others).

The beta-blocker is propranolol which may be present in the L or D-propranolol form, or racemate, or in one of its pharmaceutically acceptable salts such as the hydrochloride salt.

The beta-blocker propranolol that is used will be present in the solution in an amount of from 0.01 to 20% w/V, for example 0.01 to 5% w/V, e.g. 0.01 to 1%w/V, e.g. 0.01 to 0.5% w/V, for example from 0.05 to 10% w/V and e.g. from 0.1 to 5 % w/V and for example from 0.1 to 1% w/V.

The aqueous solutions of the invention may further contain a buffer in order to provide the appropriate pH to the solution for the beta-blocker. For example, an appropriate pH for propranolol would be of about 2 to 6, for example 2 to 5.5, e.g. 2 to 5, e.g. 2.5 to 4, e.g. 3.0 to 5.0, provided by the use of a suitable buffering agent such as mineral acids such as hydrochloric acid or organic acids such as citric acid, or buffer mixtures such as citric acid/sodium citrate, acetic acid/sodium acetate.

The present invention is based on the surprising and unexpected discovery that aqueous solutions comprising a beta-blocker such as propranolol could be free of alcohol and any preservative agents although such compositions are intended for a pharmaceutical use. Particularly, the present invention provides aqueous solutions of beta-blockers such as propranolol that are substantially free of any preservative agents and alcohol. These solutions are suitable for multiple use, i.e. the solutions can be stored between different administrations steps where administration must be repeated, e.g. once or twice daily.

Because paediatric products are intended to patients who have limited immune defences, the tendency is to take all the precautionary measures in order to avoid any bacterial infection. It is thus particularly surprising that aqueous solutions being substantially free of aromatic preservatives (and in some cases substantially free of preservatives at all) and alcohol could be safely preserved and suitably administered to infants.

The term "aromatic preservative agent" encompasses (besides the propranolol itself), any agent that is usually added to avoid the growth of microorganisms or avoid undesirable side reactions. Examples of preservative agents that are conventionally met, are benzalkonium chloride, benzethonium chloride, benzoic acid, benzyl alcohol, cetylpyridinium chloride, phenoxyethanol, cresol, phenol, phenylethyl alcohol, phenylmercuric acetate or nitrate, sodium benzoate, thimerosal, thymol compounds or compounds of the parabens family such as methylparaben, ethylparaben, propylparaben, butylparaben, and isobutylparaben.
The term "alcoholic" refers to alcohols for example ethanol.

By avoiding aromatic preservatives, the aqueous solutions according to the present invention may comprise preservative agents that are safe for paediatric patients. This includes for example the use of chlorobutanol, dehydroacetic acid, sodium dehydroacetate, propionic acid, propionate salt, such as sodium propionate or potassium propionate, sorbic acid, sorbate salt such as potassium sorbate or sodium sorbate, or mixtures thereof.

The term "substantially free" implies that the solutions within the scope of the invention should be as free of aromatic preservative agent as it is practically and realistically feasible. This covers notably compositions comprising less than 0.01w/V% of the compound of interest.
Compositions according to the invention appeared to remain stable, preserved from contamination and thus suitable for a paediatric use.
The aqueous solutions of the invention also include one or more sweetening agents that are specifically of a non-sugar type. These are incorporated into the formulation in order to enhance the taste of the dosage forms and provide compositions that will be edible by the young patients. Sweeteners notably mask the bitterness and anaesthesic effect of propranolol.

Indeed, paediatric patients are able to recognize sweetness from an early stage of life but also to recognize sweet taste in mixtures and estimate the strength or degree of sweetness.
Any artificial or organic non-sugar type sweetener may be used besides those that are unsuitable for paediatric products. The term "sugar type sweetener" encompasses monosaccharides or disaccharides such as e.g. sucrose, fructose, glucose or dextrose. Sucrose, which is the most commonly used sweetening agent is a disacharride that is hydrolysed in the intestine to the absorbable monosacharride fructose and glucose.
The term "non-sugar" encompasses artificial sweetening agents that reduce the caloric intake such as, for instance, any of saccharin, saccharin salts, e.g., sodium saccharin, calcium saccharin, sucralose, potassium acetosulfam, stevioside, steviol, mannitol, erythritol, lactitol, maltitol, alitame, miraculin, monellin, and thaumatin.
Non sugar type sweetening agents are preferred in the instant invention since some patients may suffer hereditary fructose intolerance or diabetes, while it has been established that sucrose causes a decrease in dental plaque pH, the dissolution of tooth enamel, and promotes dental caries. It has also been observed that fructose causes laxative effects at high doses.
A most preferred non sugar sweetening agent is saccharine and for example saccharine sodium or calcium salt.
Aspartame, xylitol and sorbitol are preferably excluded from the instant solutions. Both sorbitol and xylitol may cause osmotic diarrhea while sorbitol is degraded into fructose and thus represents potential risks to patients who have hereditary fructose intolerance and hypoglycaemia. Aspartame, may be harmful in patients with phenylketonuria and is usually contra-indicated in homozygous automosal recessive patients. Therefore, these components are preferably excluded from the present invention.
The sweetening agent should be present in an amount that is in accordance with safety recommendations and that provides a taste-masking efficacy but in a sufficiently low amount no to develop well-known bitter taste, as it is the case for saccharine.
Amounts of sweetening agents may be typically comprised in the range of from 0.01 to 5% w/V, for example 0.05 to 1% w/V and for example from 0.05 to 0.5 % w/V and for example from 0.05 to 0.2%w/V.

In certain embodiments, the present invention may further comprise flavouring agents which have the advantage of enhancing the potential acceptance of the composition by the child, but also have the advantage of providing a taste-masking effect for the other excipients that are found in the composition. The appropriate type and amount of flavour depends on social and cultural factors, such as food selection of adults which may have affected the flavour preferences of children.
Suitable flavours can be organic or artificial flavours it they are food grade certified by the manufacturer and have approved by the competent regulatory authorities in order to be adapted for the instant embodiment.
Examples of appropriate flavours would then be any that tastes cherry, lemon, lime, mandarin, orange, tangerine, mint, strawberry, banana, caramel, liquorice, passion-fruit, peach, raspberry, tutti-frutti, grapefruit, vanilla, cream, chocolate, grape or mixture thereof. Considering the bitter taste of beta-blockers, and more particularly of propranolol, the red or yellow fruit flavours as well as the chocolate, vanilla, caramel are preferred.
For example the strawberry and vanilla flavours appeared to be particularly accepted by the patients. Furthermore, the vanilla flavour has also recognized preservative effects and enhances the sweetness of the formulation, while the strawberry is particularly suitable to mask the bitterness of the beta-blocker such as propranolol.
Amounts of flavouring agents may typically be comprised in the range of from 0 to 5% w/V, for example from 0.01 to 1 w/V, e.g. 0.01-0.5% w/V and for example from 0.05 to 1% w/V and e.g. from 0.05 to 0.5 % w/V %.
It should be noted that some flavouring agents may be dissolved in propylene glycol. Products containing high levels of propylene glycol should not be administered to paediatric patients below the age of 4 years (EMEA/CHMP/PEG/194810/2005). According to the present invention, flavouring agents provide an amount of propylene glycol which is below this level. For example, an amount of propylene glycol as low as of 0.23% w/V is acceptable for paediatric patients and is obtained by the use of strawberry and vanilla flavours in a respective amount of 0.11% w/V and 0.21%w/V as disclosed in the formulations of example 1.

According to another embodiment, compositions of the present invention may further comprise a viscosity increasing agent in order to enhance the palatability of the solution, emulsion or suspension. A thickened liquid may also be particularly convenient and adapted to be administered to infants to avoid spillage of solutions. Furthermore, it also has the advantage to facilitate the manipulation of the solution out of the container when using a pipette or an oral syringe
Examples of thickener agents that can be used in the present invention are the celluloses derivatives such as hydroxyethylcellulose, hydroxypropylcellulose, hydroxypropylmethylcellulose, methylcellulose, gums, guar gum, tragacanthe gum, acacia gum, xanthan gum, gelane gum, alginic derivatives, alginic acid, sodium alginate, polyvinylpyrrolidone, silicates, bentonite, laponite, veegum. Poloxamers can also show thickening properties. Preferred viscosity increasing agents is hydroxyethylcellulose since it is particularly suitable for aqueous solutions.
Typically, the amount of viscosity increasing agent may be comprised from 0 to 15% w/V, for example from 0.1 to 10% w/V, e.g. from 0.1 to 5 % w/V % and e.g. from 0.1 to 0.5%w/V.
The compositions of the instant invention can further comprise any other excipient that would be suitable for a pharmaceutical use. This includes for example, solubility enhancing agents, buffers, colouring agents emulsifiers and solvents. Further examples of conventional buffers and excipients that may be considered by a person skilled in the art can be found in 'Handbook of Pharmaceutical Excipients'; Ed. A.H. Kibbe, 3rd Ed., American Pharmaceutical Association, USA and Pharmaceutical Press UK, 2000.

The compositions and formulations within the scope of the invention may conveniently be prepared from conventional processes, involving the dissolution of propranolol into a suitable solvent e.g. purified water.
Suitable processes may more conveniently be conducted according to the quality standards that are defined for example in the Note for Guidance on process for validation (EMEA/CVMP/598/99) and annex II to the Note for Guidance on process validation (CHMP/QWP/848/99) in order to obtain validation of the manufacturing process in a marketing authorisation.

According to another aspect, disclosed is a device comprising a container with the aqueous solutions according to the invention and a graduated or scaled marked pipette or oral syringe indicating doses in relation to the body weight of the patient. This pipette will for example indicate doses in relation to the body weight of the patient. This device will be used for the storage and convenient delivery of the liquid into the buccal cavity of the child.

A plastic material is particularly suited for the pipette. Because it is usually difficult to evaluate the appropriate dose that should be administered without risks of overdoses (especially in the case where the active ingredient is propranolol), this container and graduated pipette provides visual indications that allows an exact dosage directly based on the weight of the patient.

The container can be made out of a plastic or glass bottle, glass, for example amber glass in order to avoid unnecessary degradation from light of the propranolol during storage.

Since the product is intended for paediatric patients, the container should ideally be closed with a child resistant closure in order to render the content of the container inaccessible to infants.

### Example 1: Aqueous solutions within the scope of the invention.

| | 1 | 2 | 3 | 4 |
|---|---|---|---|---|
| propranolol HCl | 0.143 g | 0.285 g | 0.428 | 0.57 g |
| *corresponding to propranolol* | *0.125 g* | *0.250* g | *0.375* g | *0.50* g |
| hydroxyethyl cellulose | 0.35 g | | | 0.35 g |
| sodium Saccharin | 0.15 g | | | 0.15 g |
| sodium propionate | 0.10 g | | | - |
| strawberry flavour | 0.11 g | | | 0.11 g |
| vanilla flavour | 0.21 g | | | 0.21 g |
| (propylene glycol from flavours) | 0.23 g | | | 0.23 g |
| monohydrated citric acid | pH=3 | | | pH=3 |
| Purified water | QSP 100 ml | | | QSP 100 ml |

The solutions were prepared by placing most of the purified water which is necessary into an appropriate stainless steel tank. Under stirring, the following components were added and dissolved: a. hydroxyethylcellulose, b. saccharin sodium, c. propranolol hydrochloride, d. strawberry flavour, e. vanilla flavour. The pH of the solution was then adjusted to the pH value of 3.0 with an aqueous solution of monohydrated citric acid. Finally, the bulk was brought to the final volume with the remaining amount of water and well mixed.

Once filtered, the prepared solutions can be filled into the amber glass bottles and closed with a child resistant cap.

### Example 2: Microbiological data

The following formulations were tested to different strains in order to determine their microbiological activity and resistance.

| Components | For 100 ml | | |
|---|---|---|---|
| | Solution A | Solution B | Solution E |
| Propranolol hydrochloride | 0.50 g | 0.50 g | 0.50 g |
| hydroxyethylcellulose for e.g. Natrosol HHX250 | 0.35g | 0.35g | 0.35 g |
| Sodium Saccharine | 0.15g | 0.15g | 0.15 g |
| Hydrophilic flavour | 0.5g (vanilla) | 0.5g (caramel/vanilla) | 0.50 g (strawberry) |
| Citric acid monohydrated | Qs pH =5.00 | Qs pH =3.00 | Qs pH = 3.0 |
| Preservative | No | Methylparaben 0.1 g | No |
| Purified water | Qs to 100 ml | Qs to 100 ml | Qs to 100 ml |

In the following tests, each test products were inoculated with 200 µl of each test strain diluted in tryptone salt. Microbiological countings were performed at day 0, 14 and 28, after that the samples were neutralized in 9 ml of a neutralizing solution (10 minutes) comprising tween 80 (10%), lecithin (2%) Saponin thiosulfate (0.5%) and sterile distilled water, and that 1 ml of that neutralized mixture was transferred in Petri dishes and covered with 15 ml of agar medium (Medium based on trypcase soy agar in case for aerobic bacteria and medium based on sabouraud dextrosed agar for yeasts and moulds). Countings were achieved after 24-48 hours incubation at 32.5°C ± 2.5°C for aerobic bacteria, and 48-72 hours at 22.5°C ± 2.5°C for yeasts and moulds. For each tested solution, the following results indicate the number of colony forming unit per ml, i.e. CFU/ml of test product and the ratio of reduction (in log) in comparison to the number of micro-organisms introduced in the inoculum. The results are expressed in the below table in CFU/ml of test product and in logarithmic reduction

The tested samples were all tested to the following micro-organisms: *Staphylococcus aureus, Pseudomonas aeruginosa, Escherichia coli, Candida albicans, and Aspergillus Niger.*

For each tested solution, the following results indicate the number of recovered micro-organisms towards the different strains, as well as the ration of reduction (in log) in comparison to the number of micro-organisms introduced in the inoculum. Typically, a reduction of from > 4.7 log or more indicates the complete disappearance of micro-organisms, due to the sensibility of the apparels.

**Solution A.**

| strain | Inoculum | D0 | 14 days | | 28 days | | Accordance with Eur Ph. criteria |
|---|---|---|---|---|---|---|---|
| S. aureus | 9.8.10⁵ | 1.10 | < 10 | > 5 log | <10 | > 5 log | + |
| P. aeruginosa | 5.10⁵ | <10 | < 10 | > 4.7 log | < 10 | > 4.7 log | + |
| E. coli | 1.3.10⁶ | 2.8.10⁵ | < 10 | > 5 log | < 10 | > 5 log | + |
| C. albicans | | 1.7.10⁵ | < 10 | > 4 log | < 10 | > 4 log | + |
| A. Niger | 1.3.10⁵ | 2.9.10⁵ | 6.8.10³ | 1.3 log | 5.3.10³ | 1.4 log | + |

**Solution B.**

| strain | Inoculum | D0 | 14 days | | 28 days | | Accordance with Eur Ph. criteria |
|---|---|---|---|---|---|---|---|
| S. aureus | 9.8.10⁵ | 8.6.10⁶ | < 10 | > 5 log | < 10 | > 5 log | + |
| P. aeruginosa | 5.10⁵ | < 10 | < 10 | > 4.7 log | < 10 | > 4.7 log | + |
| E. coli | 1.3.10⁶ | 8.5.10⁵ | < 10 | > 5 log | < 10 | > 5 log | + |
| C. albicans | 0.9.10⁵ | 1.3.10⁵ | < 10 | > 4 log | < 10 | > 4 log | + |
| A. Niger | 1.3.10⁵ | 3.4.10⁵ | 4.1.10³ | 1.5 log | 1.8.10³ | 1.9 log | + |

**Solution E:**

| strain | log R after 14 days | log R after 28 days | Accordance with Eur Ph. criteria | Accordance with US Ph. criteria |
|---|---|---|---|---|
| *S*. *aureus* | > 5 log | > 5 log | + | + |
| *P*. *aeruginosa* | > 4.7 log | > 4.7 log | + | + |
| *E. coli* | > 5 log | > 5 log | + | + |
| *C*. *albicans* | > 4 log | > 4 log | + | + |
| *A. Niger* | 2 log | 0.4 log | - | + |

Recommended criteria (European Pharmacopoeia 6th edition (2008)) (Chap.5.1.3) for oral formulations.

| | T0 + 14 days | T0 + 28 days |
|---|---|---|
| Bacteria | 3 log | No increase |
| Moulds and yeasts | 1 log | |

Recommended criteria (US Pharmacopeia) for oral formulations

| | T0 + 14 days | T0 + 28 days |
|---|---|---|
| Bacteria | of 1 log | No increase |
| Moulds and yeasts | No increase | |

Thus, it clearly appears from the recommended criteria (European Pharmacopeia Chap 5.1.3) for oral formulations that the formulations of the invention remain adapted against microbiological development. The formulation with strawberry flavour as only flavouring agent at the concentration of the present example doesn't meet the requirement of the test according to the Ph. Eur. Criteria (5.1.3) for oral use.

### Example 3:

Based on a similar assay as in example 2, the preservative effect of propranolol was demonstrated by testing the following compositions, Solution C and Solution D, to the previous strains.

| Components | For 100 ml | |
|---|---|---|
| | Solution C | Solution D |
| Propranolol hydrochloride | Placebo (does not contain propranolol HCl) | 0.50 g |
| hydroxyethylcellulose for e.g. Natrosol HHX250 | 0.35 g | 0.35 g |
| Hydrphilic flavour | 0.11 g strawberry 0.21 g Vanilla | 0.11 g strawberry 0.21g Vanilla |
| Sodium Saccharine | 0.15 g- | 0.15 g- |
| Preservative | No | No |
| Citric acid monohydrated | Qs pH =2.84 or 3.0 | Qs pH =3.0 |
| Purified water | Qs 100 ml | Qs 100 ml |

**Solution C :**

| Strain | log R after 14 days | log R after 28 days | Accordance with Ph. Eur. criteria | Accordance with USP criteria |
|---|---|---|---|---|
| *S*. *aureus* | > 4.9 log | > 4.9 log | + | + |
| *P. aeruginosa* | > 4.8 log | > 4.8 log | + | + |
| *E. coli* | > 5 log | > 5 log | + | + |
| *C*. *albicans* | 1.6 log | 1.6 log | + | + |
| *A. Niger* | 0.6 log | 1.5 log | - | + |

**Solution D:**

| Strain | log R after 14 days | log R after 28 days | Accordance with Ph. Eur. criteria | Accordance with USP criteria |
|---|---|---|---|---|
| *S*. *aureus* | > 4.9 log | > 4.9 log | + | + |
| *P. aeruginosa* | > 4.8 log | > 4.8 log | + | + |
| *E. coli* | > 5 log | > 5 log | + | + |
| *C. albicans* | > 4.9 log | > 4.9 log | + | + |
| *A. Niger* | 3.2 log | 2.6 log | + | + |

The results indicate that the formulation with the placebo and without preservative agent was not acceptable under any of the US or the European Pharmacopeia recommendations after 14 days and 28 days. The composition containing propranolol, e.g. 0.50 g/100 ml without preservatives, however fulfilled the criteria even after 28 days.

For each tested solution, the results indicate the ratio of reduction (in log) in comparison to the number of micro-organisms introduced in the inoculum.

### Example 4: Based on a similar assay as in example 2, assessment of the preservative effect of propranolol 0.125%.

| | Solution F | Solution G |
|---|---|---|
| Propranolol hydrochloride 0.143 g corresponding to | 0.125g | 0.125 g of propranolol |
| hydroxyethylcellulose, for example Natrosol HHX250 | 0.35 g | 0.35 g |
| Saccharin sodium | 0.15 g | 0.15 g |
| Hydrophilic flavour | 0.11 g strawberry | 0.11 g strawberry |
| | 0.21 g Vanilla | 0.21 g Vanilla |
| Preservative, for example propionate | No | 0.1 g |
| Citric acid monohydrate | Qs pH = 3.0 | Qs pH =3.07 |
| purified water | Qs to 100 ml | Qs to 100 ml |

**Solution F:**

| Strain | log R after 14 days | log R after 28 days | Accordance with Ph. Eur. criteria | Accordance with USP criteria |
|---|---|---|---|---|
| *S*. *aureus* | > 4.9 log | > 4.9 log | + | + |
| *P*. *aeruginosa* | > 4.8 log | > 4.8 log | + | + |
| *E. coli* | > 5 log | > 5 log | + | + |
| *C. albicans* | 3.4 log | > 4.9 log | + | + |
| *A. Niger* | 0.7 log | 0.3 log | - | + |

**Solution G:**

| Strain | log R after 14 days | log R after 28 days | Accordance with Ph. Eur. criteria | Accordance with USP criteria |
|---|---|---|---|---|
| *S. aureus* | > 4.9 log | > 4.9 log | + | + |
| *P*. *aeruginosa* | > 4.8 log | > 4.8 log | + | + |
| *E. coli* | > 5 log | > 5 log | + | + |
| *C*. *albicans* | > 4.5 log | > 4.5 log | + | + |
| *A. Niger* | > 4.5log | > 4.5 log | + | + |

The results indicate that solution F without preservative agent was not acceptable under the European Pharmacopeia recommendations after 14 and 28 days.

### Example 5: Based on a similar assay as in example 2, assessment of the preservative effect of propranolol 0.250 and 0.375 %.

| | Solution H | Solution I |
|---|---|---|
| Propranolol hydrochloride | 0.285 g | 0.428 |
| *Corresponding to propranolol* | 0.250 g | 0.375 g |
| Saccharin sodium | 0.15 g | 0.15 g |
| Hydrophilic flavour | 0.11 g strawberry | 0.11 g strawberry |
| | 0.21 g Vanilla | 0.21 g Vanilla |
| Preservative, for example propionate | No | No |
| Citric acid monohydrate | QspH=3.0 | Qs pH =3.07 |
| purified water | Qs to 100 ml | Qs to 100 ml |

**Solution H:**

| Strain | log R after 14 days | log R after 28 days | Accordance with Ph. Eur. criteria | Accordance with USP criteria |
|---|---|---|---|---|
| *S*. *aureus* | > 5 log | > 5 log | + | + |
| *P. aeruginosa* | > 4.9 log | > 4.9 log | + | + |
| *E. coli* | > 4.9 log | > 4.9 log | + | + |
| *C. albicans* | > 4.9 log | > 4.9 log | + | + |
| *A. Niger* | 1.6 log | 2.2 log | + | + |

**Solution I :**

| Strain | log R after 14 days | log R after 28 days | Accordance with Ph. Eur. criteria | Accordance with USP criteria |
|---|---|---|---|---|
| *S. aureus* | > 5 log | > 5 log | + | + |
| *P. aeruginosa* | > 4.9 log | > 4.9 log | + | + |
| *E. coli* | > 4.9 log | > 4.9 log | + | + |
| *C. albicans* | > 4.9 log | > 4.9 log | + | + |
| *A. Niger* | 1.9 log | 2.5 log | + | + |

The results indicate that solutions H and I without preservative agent are acceptable under the European Pharmacopeia recommendations after 14 and 28 days.

### Example 6: Based on a similar assay as in example 2, assessment of the preservative effect of propranolol 0.375 % with or without preservative in the presence of hydroxyethyl cellulose.

| | Solution J | Solution K |
|---|---|---|
| Propranolol hydrochloride 0.428 g corresponding to propranolol | 0. 375 g | 0.375 g |
| hydroxyethylcellulose, for example Natrosol HHX250 | 0.35 g | 0.35 g |
| Saccharin sodium | 0.15 g | 0.15 g |
| Hydrophilic flavour | 0.11 g strawberry | 0.11 g strawberry |
| | 0.21 g Vanilla | 0.21 g Vanilla |
| Preservative, for example propionate | No | 0.1 g |
| Citric acid monohydrate | Qs pH = 2-6 | Qs pH = 2-6 |
| purified water | Qs to 100 ml | Qs to 100 ml |

### Example 7: Based on a similar assay as in example 2, assessment of the preservative effect of propranolol 0.375 % with different concentrations of preservatives for example 0.025, 0.05, 0.075%; for example of propionate.

### Example 8: Normal multi-use test.

Solution D was tested as follows: pipette was introduced and soaked into the bottle comprising the solution of the invention. The pipette was rinsed with tap water and left on the table until the next use. This operation was performed twice a day over a period of one month. In a similar example, the use was performed with a contaminated pipette.

Both uses fulfil the criteria of the Pharmacopoeia.

This test is performed to mimic the real life conditions of use of the solution according to the invention. A pipette is used to measure the amount to administer to the patient and optionally, said amount will be administered directly into the mouth of the patient and optionally after said use the pipette is left on the table without a rinsing step. This test also mimics the situation where the pipette is contaminated with microorganisms, during its use, not rinsed and introduced again in the solution. The solution can thus be contaminated with the microorganisms.

The present test of Example 8 fulfilling the criteria of the Pharmacopoeia is thus indicating that the solution of the invention is a multi-use solution, for example up to a 100 uses.

### Example 9: Clinical Protocol

1) Diagnosis and criteria for inclusion: A subject is eligible if he/she meets all of the following criteria:
   a) Written informed consents for study participation and the use of the subject's images are obtained from the subject's legal parental custodian(s) prior to performing any study procedures.,
   b) The subject is 35 to 150 days old, inclusive, at inclusion,
   c) A facial proliferating infantile hemangioma (IH), for example, a large facial proliferating IH with largest diameter of at least 1.5 cm requiring systemic therapy is present.
2) Mode of administration: Administration of propranolol oral solution according to Example 1 above, formula 1, 2 and 3 of example 1, twice daily (morning and late afternoon) for 3 or 6 months
3) Titration procedure: D0 1 mg/kg/day, D7 increase to 2 mg/kg/day (for the 3 mg/kg/day arm), D14 increase to 3 mg/kg/day (for the 3 mg/kg/day arm).
4) Treatment duration: Propranolol is administered for 3 months or 6 months, depending on the assigned regimen.
5) Evaluation criteria:
   The primary efficacy criterion is the evolution of target IH from baseline to W24.

## Claims

1. An aqueous ethyl alcohol free paediatric solution comprising propranolol or pharmaceutically acceptable salts thereof, a non sugar type sweetener and less than 0.01 w/V% of any preservative agents, other than propranolol itself, for use as a medicament in the treatment of hemangiomas, wherein the solution is a multi-use solution.

2. The solution according to claim 1, for use as a medicament in the treatment of capillary hemangiomas.

3. The solution according to claim 1, for use as a medicament in the treatment of capillary infantile hemangiomas.

4. The solution according to anyone of claims 1 to 3, wherein the propranolol or pharmaceutically acceptable salts thereof is present in an amount of from 0.01 to 5% w/V, for example from 0.01 to 1% w/V.

5. The solution according to anyone of claims 1 to 4, further comprising at least one flavouring agent and/or at least one viscosity increasing agent.

6. The solution according to claim 5, wherein the at least one flavouring agent is selected from any of the cherry, lemon, lime, mandarin, orange, tangerine, mint, strawberry, banana, caramel, liquorice, passion-fruit, peach, raspberry, tutti-frutti, grapefruit, vanilla, cream, chocolate, grape flavour or mixture thereof, for example selected from the vanilla and strawberry flavour, and is present in an amount of from 0 to 5% w/V, e.g. of from 0.01 to 1% w/V and e.g. of from 0.01 to 0.5 % w/V.

7. The solution according to claim 6, wherein the at least one flavouring agent is vanilla in an amount of from 0.01 to 0.5% w/V.

8. The solution according to any one of claims 5 to 7, wherein the at least one viscosity increasing agent is selected from cellulose derivatives, hydroxyethylcellulose, hydroxypropylcellulose, hydroxypropylmethylcellulose, or methylcellulose, poloxamers, from gums, guar gum, tragacanthe gum, acacia gum, xanthane gum, gelane gums, alginic derivatives, alginic acid, sodium alginate, polyvinylpyrrolidone, from silicates, bentonite, laponite, veegum, more particularly from non-ionic poloxamers, polyvinylpyrrolidone and cellulose ethers, in an amount of from 0 to 15% w/V, e.g. of from 0.1 to 10% w/V, e.g. of from 0.1 to 5 % w/V and e.g. of from 0.1 to 0.5% w/V.

9. The solution according to claim 8 wherein the at least one viscosity increasing agent is hydroxyethylcellulose.

10. The solution according to anyone of claims 1 to 9, further comprising a pH regulator agent or buffer, and wherein the pH is comprised between 2 and 6, for example 2 and 5.5, for example between 3.0 and 5.0, e.g. 2.0 and 5.0, e.g. 2.5 and 4.

11. The solution according to anyone of claims 1 to 10, wherein the at least one non-sugar type sweetener is selected from saccharin, saccharin salts, sodium saccharin, calcium saccharin, sucralose, potassium acetosulfam, stevioside, steviol, mannitol, erythritol, lactitol, maltitol, alitame, miraculin, monellin, thaumatin, and mixture thereof, and for example selected from saccharin sodium, and is in an amount of from 0.05 to 0.5% w/V.

12. The solution according to claim 6 wherein propranolol is present in an amount of 0.250 to 1% w/V and vanilla is present in an amount of 0.01 to 1% w/V and wherein said aqueous solution does not contain a preservative agent.

13. The solution according to anyone of claims 1 to 12, wherein the propranolol or pharmaceutically acceptable salts thereof is propranolol hydrochloride in an amount of 0.428 or 0.57 % w/V wherein the at least one sweetener is saccharin sodium in an amount of 0.15% w/V, wherein the at least one flavouring agent is a mixture of vanilla and strawberry flavour in an amount of 0.32% w/V the at least one viscosity increasing agent is hydroxyethylcellulose in an amount of 0.35 % w/V.

## Patentansprüche

1. Eine wässrige alkoholfreie pädiatrische Lösung mit Propanolol oder pharmazeutisch verträglichen Salzen davon, ein Süßstoff des Typs nicht-Zucker und weniger als 0,01 Gew.-%/V% an Konservierungsmitteln außer dem Propanolol selbst zur Verwendung als Medikament zur Behandlung von Hämangiomen, wobei die Lösung eine Mehrfachnutzung Lösung ist.

2. Die Lösung nach Anspruch 1, zur Verwendung als Medikament zur Behandlung von kapillären Hämangiomen.

3. Die Lösung nach Anspruch 1, zur Verwendung als Medikament in der Behandlung von infantilen kapillären Hämangiomen.

4. Die Lösung nach einem der Ansprüche 1 bis 3, wobei das Propanolol oder pharmazeutisch verträgliche Salze davon in einer Menge von 0,01 bis 5 Gew.%/V, zum Beispiel von 0,01 bis 1 Gew.-%/V vorliegt.

5. Die Lösung nach einem der Ansprüche 1 bis 4 ferner umfassend mindestens einen Aromastoff, und/oder mindestens ein die Viskosität erhöhendes Mittel.

6. Die Lösung nach Anspruch 5, wobei der mindestens eine Aromastoff aus einem der Kirsche, Zitrone, Limette, Mandarine, Orange, Tangerine, Minze, Erdbeere, Banane, Karamell, Lakritz, Passionsfrucht, Pfirsich, Himbeere, Tutti-Frutti, Grapefruit, Vanille, Sahne, Schokolade, Traubengeschmack oder einer Mischung davon, beispielsweise von Vanille und Erdbeeraroma ausgewählt ist und beispielsweise in einer Menge von 0 bis 5 Gew.-%/V, z.B. von 0,01 bis 1% Gew.-%/V und z.B. von 0,01 bis 0,5 Gew.-%/V vorliegt.

7. Die Lösung nach Anspruch 6, bei der mindestens ein Aromastoff Vanille in einer Menge von 0,01 bis 0,5 Gew.-%/V ist.

8. Die Lösung nach einem der Ansprüche 5 bis 7, wobei das zumindest eine die Viskosität erhöhende Mittel aus Zellulosederivaten, Hydroxyethylzellulose, Hydroxypropylzellulose, Hydroxypropylmethylzellulose oder Methylzellulose, Poloxamere, aus Gummi, Guargummi, Traganthgummi, Akaziengummi, Xanthangummi, Gelangummi, Alginsäure-Derivaten, Alginsäure, Natriumalginat, Polyvinylpyrrolidon, aus Silikaten, Bentonit, Laponit, Veegum, insbesondere aus nicht ionischen Poloxameren, Polyvinylpyrrolidonen und Zelluloseethern beispielsweise in einer Menge von 0 bis 15 % Gew.-%/V, z.B. von 0,1 bis 10 % Gew.-%/V, z.B. von 0,1 bis 5% Gew.-%/V und z.B. von 0,1 bis 0,5% Gew.-%/V.

9. Die Lösung nach Anspruch 8, wobei das zumindest eine die Viskosität erhöhende Mittel Hydroxyethylzellulose ist.

10. Die Lösung nach einem der Ansprüche 1 bis 9, ferner umfassend ein pH-Einstellmittel oder Puffer, und wobei der pH-Wert zwischen 2 und 6 liegt, zum Beispiel zwischen 2 und 5,5 und zum Beispiel zwischen 3,0 und 5,0 und zum Beispiel zwischen 2,0 und 5,0 und zum Beispiel zwischen 2,5 und 4.

11. Die Lösung nach einem der Ansprüche 1 bis 10, wobei der mindestens eine Süßstoff des Typs Nicht-Zucker aus Saccharin, Saccharinsalzen, Natriumsaccharin, Calciumsaccharin, Sucralose, Kalium-Acesulfam, Steviosid, Steviol, Mannitol, Erythritol, Lactitol, Maltitol, Alitam, Miraculin , Monellin, Thaumatin und Mischungen davon, und beispielsweise aus Natriumsaccharin ausgewählt wird und in einer Menge von 0,05 bis 0,5 Gew.-%/V vorliegt.

12. Die Lösung nach Anspruch 6, wobei Propranolol in einer Menge von 0,250 bis 1 Gew.-%/V vorliegt und Vanille in einer Menge von 0,01 bis 1 % Gew.-%/V vorliegt und die wässrige Lösung kein Konservierungsmittel enthält.

13. Die Zusammensetzung nach einem der Ansprüche 1 bis 12, wobei das Propranolol oder eines der pharmazeutisch verträglichen Salze davon Propanolol-Hydrochlorid ist in einer Menge von 0,428 oder 0,57 Gew.-%/V, wobei der zumindest eine Süßstoff Natriumsaccharin in einer Menge von 0,15 Gew.-%/V ist, wobei der mindestens eine Geschmacksstoff eine Mischung von Vanille- und Erdbeeraroma in einer Menge von 0,32 Gew.-%/V ist, wobei das mindestens eine die Viskosität erhöhende Mittel Hydroxyethylzellulose in einer Menge von 0,35 Gew.-%/V ist.

## Revendications

1. Solution pédiatrique aqueuse dépourvue d'alcool éthylique comprenant du propranolol ou un de ses sels pharmaceutiquement acceptables, un édulcorant de type non sucré et moins de 0,01% p/V d'agents conservateurs, autres que le propranolol lui-même, pour son utilisation comme médicament dans le traitement des hémangiomes, dans laquelle la solution est une solution multi-usage.

2. Solution selon la revendication 1, pour son utilisation comme médicament dans le traitement des hémangiomes capillaires.

3. Solution selon la revendication 1, pour son utilisation comme médicament dans le traitement des hémangiomes capillaires infantiles.

4. Solution selon l'une quelconque des revendications 1 à 3, dans laquelle le propranolol ou un de ses sels pharmaceutiquement acceptables est présent en une quantité allant de 0,01 à 5% p/V, par exemple de 0,01 à 1% p/V.

5. Solution selon l'une quelconque des revendications 1 à 4, comprenant en outre au moins un agent aromatisant et/ou au moins un agent augmentant la viscosité.

6. Solution selon la revendication 5, dans laquelle ledit au moins un agent aromatisant est sélectionné parmi l'un des arômes de cerise, citron, citron vert, mandarine, orange, tangerine, menthe, fraise, banane, caramel, réglisse, fruit de la passion, pêche, framboise, tutti-fruti, pamplemousse, vanille, crème, chocolat, raisin ou un mélange de ceux-ci, et est présent en une quantité allant de 0 à 5% p/V, par exemple de 0,01 à 1% p/V et par exemple allant de 0,01 à 0,5% p/V.

7. Solution selon la revendication 6, dans laquelle l'au moins un agent aromatisant est la vanille en une quantité allant de 0,01 à 0,5 % p/V.

8. Solution selon l'une quelconque des revendications 5 à 7, dans laquelle l'au moins un agent augmentant la viscosité est sélectionné parmi les dérivés de cellulose, l'hydroxyéthylcellulose, l'hydroxypropylcellulose, l'hydroxypropylméthylcellulose, ou la méthylcellulose, les poloxamères, parmi les gommes, gomme de guar, gomme tragacanthe, gomme d'acacia, gomme xanthane, gommes gélanes, dérivés alginiques, l'acide alginique, l'alginate de sodium, la polyvinylpyrrolidone, parmi les silicates, la bentonite, la laponite, le veegum, plus particulièrement parmi les poloxamères non-ioniques, le polyvinylpyrrolidone et les éthers de cellulose, en une quantité allant de 0 à 15% p/V, par exemple allant de 0,1 à 10% p/V, par exemple allant de 0,1 à 5% p/V et par exemple allant de 0,1 à 0,5 % p/V.

9. Solution selon la revendication 8 dans laquelle l'au moins un agent augmentant la viscosité est l'hydroxyéthylcellulose.

10. Solution selon l'une quelconque des revendications 1 à 9, comprenant en outre un agent régulateur de pH ou un tampon, et dans laquelle le pH est compris entre 2 et 6, par exemple 2 et 5,5, par exemple entre 3,0 et 5,0, par exemple 2,0 et 5,0, par exemple 2,5 et 4.

11. Solution selon l'une quelconque des revendications 1 à 10, dans laquelle l'au moins un édulcorant de type non sucré est sélectionné parmi la saccharine, les sels de saccharine, la saccharine de sodium, la saccharine de calcium, le sucralose, l'acétosulfame de potassium, le stévioside, le stéviol, le mannitol, l'érythritol, le lactitol, le maltitol, l'alitame, la miraculine, la monelline, la thaumatine, et leurs mélanges, et par exemple sélectionné parmi la saccharine de sodium, et est en une quantité allant de 0,05 à 0,5 % p/V.

12. Solution selon la revendication 6 dans laquelle le propranolol est présent en une quantité de 0,250 à 1% p/V et la vanille est présente en une quantité de 0,01 à 1% p/V et dans laquelle ladite solution aqueuse ne contient pas d'agent conservateur.

13. Solution selon l'une quelconque des revendications 1 à 12, dans laquelle le propranolol ou ses sels pharmaceutiquement actifs est l'hydrochlorure de propranolol en une quantité de 0,428 ou 0,57% p/V dans laquelle l'au moins un édulcorant est la saccharine de sodium en une quantité de 0,15% p/V, dans laquelle l'au moins un agent aromatisant est un mélange d'arôme de vanille et de fraise en une quantité de 0,32% p/V, l'au moins un agent augmentant la viscosité est l'hydroxyéthylcellulose en une quantité de 0,35% p/V.
